# EUROPEAN PATENT APPLICATION

(11) **EP 1 950 308 A1**
(43) Date of publication of application: **30.07.2008**
(21) Application number: 06832736.0
(22) Date of filing: 16.11.2006
(51) Int. Cl.: C12P 19/04, C12N 15/09

(54) **PROCESS FOR PRODUCING CHONDROITIN**

(30) Priority: 17.11.2005 JP 2005332598
(71) Applicant: SEIKAGAKU CORPORATION, Chiyoda-ku, Tokyo 100-0005 (JP)
(72) Inventor: SUGIURA, Nobuo, Aichi-gun Aichi 480-1195 (JP); SHIMOKATA, Satoshi Seikagaku Corporation Central, Tokyo 207-0021 (JP); KIMATA, Koji, Aichi-gun Aichi 480-1195 (JP)
(74) Representative: Elsy, David
(86) International application number: PCT/JP2006/322847
(87) International publication number: WO 2007/058252

(57) **Abstract**

A method for producing a chondroitin (CH) with a desired sugar chain length; a method for producing a fraction containing a CH with a substantially single sugar chain length; etc. There is provided a method for producing a CH with desired sugar chain length, comprising alternately performing the steps (a) allowing a receptor substrate having a glucuronic acid residue at its non-reducing end (when this step is performed after the step (b), sugar chain obtained by the step (b)), an N-acetylgalactosamine donor and an N-acetylgalactosamine transferase to coexist in a reaction system and (b) allowing a receptor substrate having an N-acetylgalactosamine residue at its non-reducing end (when this step is performed after the step (a), sugar chain obtained by the step (a)), a glucuronic acid donor and a glucuronic acid transferase to coexist in a reaction system.

SEQUENCE LISTING

<110>  Seikagaku corporation

<120>  PROCESS FOR PRODUCING CHONDROITIN

<130>  SE06004PCT

<150>  JP2005-332598
<151>  2005-11-17

<160>  2

<170>  PatentIn version 3.1

<210>  1
<211>  2058
<212>  DNA
<213>  Escherichia coli

<220>
<221>  CDS
<222>  (1)..(2058)

<400>  1
atg agt att ctt aat caa gca ata aat tta tat aaa aac aaa aat tat      48
Met Ser Ile Leu Asn Gln Ala Ile Asn Leu Tyr Lys Asn Lys Asn Tyr
1               5                   10                  15
cgc caa gct tta tct ctt ttt gag aag gtt gct gaa att tat gat gtt      96
Arg Gln Ala Leu Ser Leu Phe Glu Lys Val Ala Glu Ile Tyr Asp Val
            20                  25                  30
agt tgg gtc gaa gca aat ata aaa tta tgc caa acc gca ctc aat ctt      144
Ser Trp Val Glu Ala Asn Ile Lys Leu Cys Gln Thr Ala Leu Asn Leu
        35                  40                  45
tct gaa gaa gtt gat aag tta aat cgt aaa gct gtt att gat att gat      192
Ser Glu Glu Val Asp Lys Leu Asn Arg Lys Ala Val Ile Asp Ile Asp
    50                  55                  60
gca gca aca aaa ata atg tgt tct aac gcc aaa gca att agt ctg aac      240
Ala Ala Thr Lys Ile Met Cys Ser Asn Ala Lys Ala Ile Ser Leu Asn
65                  70                  75                  80
gag gtt gaa aaa aat gaa ata ata agc aaa tac cga gaa ata acc gca      288
Glu Val Glu Lys Asn Glu Ile Ile Ser Lys Tyr Arg Glu Ile Thr Ala
                85                  90                  95
aag aaa tca gaa cgg gcg gag tta aag gaa gtc gaa ccc att cct tta      336
Lys Lys Ser Glu Arg Ala Glu Leu Lys Glu Val Glu Pro Ile Pro Leu

            100                 105                 110
gat tgg cct agt gat tta act tta ccg ccg tta cct gag agc aca aac 384
Asp Trp Pro Ser Asp Leu Thr Leu Pro Pro Leu Pro Glu Ser Thr Asn
        115                 120                 125
gat tat gtt tgg gcg ggg aaa aga aaa gag ctt gat gat tat cca aga 432
Asp Tyr Val Trp Ala Gly Lys Arg Lys Glu Leu Asp Asp Tyr Pro Arg
    130                 135                 140
aaa cag tta atc att gac ggg ctt agt att gta att cct aca tat aat 480
Lys Gln Leu Ile Ile Asp Gly Leu Ser Ile Val Ile Pro Thr Tyr Asn
145                 150                 155                 160
cga gca aaa ata ctt gca att aca ctt gct tgt ctt tgt aac caa aag 528
Arg Ala Lys Ile Leu Ala Ile Thr Leu Ala Cys Leu Cys Asn Gln Lys
                165                 170                 175
acc ata tac gac tat gaa gtt att gtt gcc gat gat gga agt aaa gaa 576
Thr Ile Tyr Asp Tyr Glu Val Ile Val Ala Asp Asp Gly Ser Lys Glu
            180                 185                 190
aat att gaa gaa ata gta aga gaa ttt gaa agt tta tta aat ata aaa 624
Asn Ile Glu Glu Ile Val Arg Glu Phe Glu Ser Leu Leu Asn Ile Lys
        195                 200                 205
tat gta cgt cag aag gat tat gga tat caa ctg tgt gct gtt aga aat 672
Tyr Val Arg Gln Lys Asp Tyr Gly Tyr Gln Leu Cys Ala Val Arg Asn
    210                 215                 220
ctt ggg ctt agg gct gca aag tat aat tat gtt gca att ctg gat tgt 720
Leu Gly Leu Arg Ala Ala Lys Tyr Asn Tyr Val Ala Ile Leu Asp Cys
225                 230                 235                 240
gat atg gct ccg aac cca cta tgg gtt cag tca tat atg gaa cta tta 768
Asp Met Ala Pro Asn Pro Leu Trp Val Gln Ser Tyr Met Glu Leu Leu
                245                 250                 255
gcg gtg gac gat aat gtt gct cta att ggc cct aga aaa tat ata gat 816
Ala Val Asp Asp Asn Val Ala Leu Ile Gly Pro Arg Lys Tyr Ile Asp
            260                 265                 270
aca agc aag cat aca tat tta gat ttc ctt tcc caa aaa tca cta ata 864
Thr Ser Lys His Thr Tyr Leu Asp Phe Leu Ser Gln Lys Ser Leu Ile
        275                 280                 285
aat gaa att cct gaa atc att act aat aat cag gtt gca ggc aag gtt 912
Asn Glu Ile Pro Glu Ile Ile Thr Asn Asn Gln Val Ala Gly Lys Val
    290                 295                 300
gag caa aac aaa tca gtt gac tgg cga ata gaa cat ttc aaa aat acc 960
Glu Gln Asn Lys Ser Val Asp Trp Arg Ile Glu His Phe Lys Asn Thr
305                 310                 315                 320
gat aat cta aga tta tgc aac aca cca ttt cga ttt ttt agc gga ggt 1008
Asp Asn Leu Arg Leu Cys Asn Thr Pro Phe Arg Phe Phe Ser Gly Gly
                325                 330                 335
aat gtc gct ttt gcg aaa aaa tgg ctt ttc cgt gca gga tgg ttt gat 1056
Asn Val Ala Phe Ala Lys Lys Trp Leu Phe Arg Ala Gly Trp Phe Asp

            340                 345                 350
gaa gag ttt acg cat tgg ggg ggg gag gat aat gag ttt gga tat cgt 1104
Glu Glu Phe Thr His Trp Gly Gly Glu Asp Asn Glu Phe Gly Tyr Arg
        355                 360                 365
ctc tac aga gaa gga tgt tac ttt cgg tct gtt gaa gga gca atg gca 1152
Leu Tyr Arg Glu Gly Cys Tyr Phe Arg Ser Val Glu Gly Ala Met Ala
    370                 375                 380
tat cat caa gaa cca ccc ggg aaa gaa aac gag acg gat cgt gcg gca 1200
Tyr His Gln Glu Pro Pro Gly Lys Glu Asn Glu Thr Asp Arg Ala Ala
385                 390                 395                 400
ggg aaa aat att act gtt caa ttg tta cag caa aaa gtt cct tat ttc 1248
Gly Lys Asn Ile Thr Val Gln Leu Leu Gln Gln Lys Val Pro Tyr Phe
                405                 410                 415
tat aga aaa aaa gaa aaa ata gaa tcc gcg aca tta aaa aga gta cca 1296
Tyr Arg Lys Lys Glu Lys Ile Glu Ser Ala Thr Leu Lys Arg Val Pro
            420                 425                 430
cta gta tct ata tat att ccc gcc tat aac tgc tct aaa tat att gtt 1344
Leu Val Ser Ile Tyr Ile Pro Ala Tyr Asn Cys Ser Lys Tyr Ile Val
        435                 440                 445
cgt tgt gtt gaa agc gcc ctt aat cag aca ata act gac tta gaa gta 1392
Arg Cys Val Glu Ser Ala Leu Asn Gln Thr Ile Thr Asp Leu Glu Val
    450                 455                 460
tgc ata tgc gat gat ggt tcc aca gat gat aca ttg cgg att ctt cag 1440
Cys Ile Cys Asp Asp Gly Ser Thr Asp Asp Thr Leu Arg Ile Leu Gln
465                 470                 475                 480
gag cat tat gca aac cat cct cga gtt cgt ttt att tca caa aaa aac 1488
Glu His Tyr Ala Asn His Pro Arg Val Arg Phe Ile Ser Gln Lys Asn
                485                 490                 495
aaa gga att ggt tca gca tct aat aca gca gtt aga ttg tgt cgg gga 1536
Lys Gly Ile Gly Ser Ala Ser Asn Thr Ala Val Arg Leu Cys Arg Gly
            500                 505                 510
ttc tat ata ggt cag tta gac tct gat gac ttt ctt gaa cca gat gct 1584
Phe Tyr Ile Gly Gln Leu Asp Ser Asp Asp Phe Leu Glu Pro Asp Ala
        515                 520                 525
gtt gaa cta tgt cta gat gaa ttt aga aaa gat cta tca ttg gca tgt 1632
Val Glu Leu Cys Leu Asp Glu Phe Arg Lys Asp Leu Ser Leu Ala Cys
    530                 535                 540
gtt tat aca act aac cgt aat ata gat cgt gaa ggt aat ttg ata tca 1680
Val Tyr Thr Thr Asn Arg Asn Ile Asp Arg Glu Gly Asn Leu Ile Ser
545                 550                 555                 560
aat ggc tat aat tgg ccc att tat tcg cga gaa aaa ctt act agt gca 1728
Asn Gly Tyr Asn Trp Pro Ile Tyr Ser Arg Glu Lys Leu Thr Ser Ala
                565                 570                 575
atg ata tgt cat cat ttc agg atg ttc aca gca aga gca tgg aac cta 1776
Met Ile Cys His His Phe Arg Met Phe Thr Ala Arg Ala Trp Asn Leu

580                 585                 590
act gaa ggt ttc aac gaa tcg atc agc aac gca gtt gat tac gat atg      1824
Thr Glu Gly Phe Asn Glu Ser Ile Ser Asn Ala Val Asp Tyr Asp Met
        595                 600                 605
tat tta aaa ctt agt gaa gtt gga ccg ttc aag cat ata aac aaa att      1872
Tyr Leu Lys Leu Ser Glu Val Gly Pro Phe Lys His Ile Asn Lys Ile
    610                 615                 620
tgt tat aat cgc gta ttg cat ggt gaa aat acg tct ata aaa aag ttg      1920
Cys Tyr Asn Arg Val Leu His Gly Glu Asn Thr Ser Ile Lys Lys Leu
625                 630                 635                 640
gat att caa aag gaa aat cat ttt aaa gtt gtt aac gaa tca tta agt      1968
Asp Ile Gln Lys Glu Asn His Phe Lys Val Val Asn Glu Ser Leu Ser
                645                 650                 655
agg cta ggc ata aaa aaa tat aaa tat tca cca tta act aat ttg aat      2016
Arg Leu Gly Ile Lys Lys Tyr Lys Tyr Ser Pro Leu Thr Asn Leu Asn
            660                 665                 670
gaa tgt aga aaa tat acc tgg gaa aaa ata gag aat gat tta              2058
Glu Cys Arg Lys Tyr Thr Trp Glu Lys Ile Glu Asn Asp Leu
        675                 680                 685

<210>  2
<211>  686
<212>  PRT
<213>  Escherichia coli

<400>  2
Met Ser Ile Leu Asn Gln Ala Ile Asn Leu Tyr Lys Asn Lys Asn Tyr
1               5                   10                  15
Arg Gln Ala Leu Ser Leu Phe Glu Lys Val Ala Glu Ile Tyr Asp Val
            20                  25                  30
Ser Trp Val Glu Ala Asn Ile Lys Leu Cys Gln Thr Ala Leu Asn Leu
        35                  40                  45
Ser Glu Glu Val Asp Lys Leu Asn Arg Lys Ala Val Ile Asp Ile Asp
    50                  55                  60
Ala Ala Thr Lys Ile Met Cys Ser Asn Ala Lys Ala Ile Ser Leu Asn
65                  70                  75                  80
Glu Val Glu Lys Asn Glu Ile Ile Ser Lys Tyr Arg Glu Ile Thr Ala
                85                  90                  95
Lys Lys Ser Glu Arg Ala Glu Leu Lys Glu Val Glu Pro Ile Pro Leu
            100                 105                 110
Asp Trp Pro Ser Asp Leu Thr Leu Pro Pro Leu Pro Glu Ser Thr Asn
        115                 120                 125
Asp Tyr Val Trp Ala Gly Lys Arg Lys Glu Leu Asp Asp Tyr Pro Arg
    130                 135                 140

Lys Gln Leu Ile Ile Asp Gly Leu Ser Ile Val Ile Pro Thr Tyr Asn
145                 150                 155                 160
Arg Ala Lys Ile Leu Ala Ile Thr Leu Ala Cys Leu Cys Asn Gln Lys
                165                 170                 175
Thr Ile Tyr Asp Tyr Glu Val Ile Val Ala Asp Asp Gly Ser Lys Glu
            180                 185                 190
Asn Ile Glu Glu Ile Val Arg Glu Phe Glu Ser Leu Leu Asn Ile Lys
        195                 200                 205
Tyr Val Arg Gln Lys Asp Tyr Gly Tyr Gln Leu Cys Ala Val Arg Asn
    210                 215                 220
Leu Gly Leu Arg Ala Ala Lys Tyr Asn Tyr Val Ala Ile Leu Asp Cys
225                 230                 235                 240
Asp Met Ala Pro Asn Pro Leu Trp Val Gln Ser Tyr Met Glu Leu Leu
                245                 250                 255
Ala Val Asp Asp Asn Val Ala Leu Ile Gly Pro Arg Lys Tyr Ile Asp
            260                 265                 270
Thr Ser Lys His Thr Tyr Leu Asp Phe Leu Ser Gln Lys Ser Leu Ile
        275                 280                 285
Asn Glu Ile Pro Glu Ile Ile Thr Asn Asn Gln Val Ala Gly Lys Val
    290                 295                 300
Glu Gln Asn Lys Ser Val Asp Trp Arg Ile Glu His Phe Lys Asn Thr
305                 310                 315                 320
Asp Asn Leu Arg Leu Cys Asn Thr Pro Phe Arg Phe Phe Ser Gly Gly
                325                 330                 335
Asn Val Ala Phe Ala Lys Lys Trp Leu Phe Arg Ala Gly Trp Phe Asp
            340                 345                 350
Glu Glu Phe Thr His Trp Gly Gly Glu Asp Asn Glu Phe Gly Tyr Arg
        355                 360                 365
Leu Tyr Arg Glu Gly Cys Tyr Phe Arg Ser Val Glu Gly Ala Met Ala
    370                 375                 380
Tyr His Gln Glu Pro Pro Gly Lys Glu Asn Glu Thr Asp Arg Ala Ala
385                 390                 395                 400
Gly Lys Asn Ile Thr Val Gln Leu Leu Gln Gln Lys Val Pro Tyr Phe
                405                 410                 415
Tyr Arg Lys Lys Glu Lys Ile Glu Ser Ala Thr Leu Lys Arg Val Pro
            420                 425                 430
Leu Val Ser Ile Tyr Ile Pro Ala Tyr Asn Cys Ser Lys Tyr Ile Val
        435                 440                 445
Arg Cys Val Glu Ser Ala Leu Asn Gln Thr Ile Thr Asp Leu Glu Val
    450                 455                 460
Cys Ile Cys Asp Asp Gly Ser Thr Asp Asp Thr Leu Arg Ile Leu Gln
465                 470                 475                 480
Glu His Tyr Ala Asn His Pro Arg Val Arg Phe Ile Ser Gln Lys Asn
                485                 490                 495
Lys Gly Ile Gly Ser Ala Ser Asn Thr Ala Val Arg Leu Cys Arg Gly

            500                 505                 510
Phe Tyr Ile Gly Gln Leu Asp Ser Asp Asp Phe Leu Glu Pro Asp Ala
        515                 520                 525
Val Glu Leu Cys Leu Asp Glu Phe Arg Lys Asp Leu Ser Leu Ala Cys
    530                 535                 540
Val Tyr Thr Thr Asn Arg Asn Ile Asp Arg Glu Gly Asn Leu Ile Ser
545                 550                 555                 560
Asn Gly Tyr Asn Trp Pro Ile Tyr Ser Arg Glu Lys Leu Thr Ser Ala
                565                 570                 575
Met Ile Cys His His Phe Arg Met Phe Thr Ala Arg Ala Trp Asn Leu
            580                 585                 590
Thr Glu Gly Phe Asn Glu Ser Ile Ser Asn Ala Val Asp Tyr Asp Met
        595                 600                 605
Tyr Leu Lys Leu Ser Glu Val Gly Pro Phe Lys His Ile Asn Lys Ile
    610                 615                 620
Cys Tyr Asn Arg Val Leu His Gly Glu Asn Thr Ser Ile Lys Lys Leu
625                 630                 635                 640
Asp Ile Gln Lys Glu Asn His Phe Lys Val Val Asn Glu Ser Leu Ser
                645                 650                 655
Arg Leu Gly Ile Lys Lys Tyr Lys Tyr Ser Pro Leu Thr Asn Leu Asn
            660                 665                 670
Glu Cys Arg Lys Tyr Thr Trp Glu Lys Ile Glu Asn Asp Leu
        675                 680                 685

## Description

### Technical Field

The present invention relates to a novel production method for a chondroitin with a desired sugar chain length, and the like.

### Background Art

First, abbreviations used in the present application document will be described.
CH: chondroitin
CS: chondroitin sulfate
HA: hyaluronic acid
GlcUA: glucuronic acid
GalNAc: N-acetylgalactosamine
GPC: gel permeation chromatography
HPLC: high performance liquid chromatography
K4CP: chondroitin polymerase derived from *Escherichia coil* K4 strain D241K: a point mutant of K4CP comprising the amino acid sequence shown in SEQ ID NO: 2 in which the aspartic acid residue of amino acid number 241 in SEQ ID NO: 2 is replaced by a lysine residue. This enzyme substantially lacks an activity to transfer GalNAc, but maintains an activity to transfer GlcUA.
D521K: a point mutant of K4CP comprising the amino acid sequence shown in SEQ ID NO: 2 in which the aspartic acid residue of amino acid number 521 in SEQ ID NO: 2 is replaced by a lysine residue. This enzyme substantially lacks an activity to transfer GlcUA, but maintains an activity to transfer GalNAc.

### MALDI-TOF-MS:

Matrix Assisted Laser Desorption/Ionization time-of-flight mass spectrometry
UDP: uridine 5'-diphosphate
PA: aminopyridine
A CH is a kind of glycosaminoglycan in which GlcUA and GalNac are linearly and alternately bound by a β1-3 linkage and a β1-4 linkage, respectively. CH is present as CS proteoglycan in a cartilage and many connective tissues in an animal body, and plays an important role in cell adherence, cell generation, cell differentiation, nerve cell extension, cartilage formation, bone formation, tissue regeneration, and the like.

Although a CH polymerase derived from an animal has been cloned, a CH cannot be synthesized by using the enzyme alone. Moreover, such an enzyme has a weak enzymatic activity, so use of such an enzyme does not sufficiently contribute to the efficient industrial production of CH sugar chains. On the other hand, K4CP has also been cloned, and it is known that the enzyme alone has an activity to synthesize a CH (Patent Document 1 and Non-patent Document 1).
K4CP is an enzyme having two active sites for sugar transfer, and allows sugar chains to extend by using UDP-GlcUA and UDP-GalNAc as donors, respectively. By changing a reaction time and an addition amount of substrates, a size (sugar chain length) of a synthesized CH can be adjusted to a certain extent. However, since the obtained fraction is a mixture containing sugar chains each having a different molecular weight, a fraction consisting a CH having only a desired sugar chain length cannot be obtained.

Each of the two active sites for sugar transfer in K4CP bears activities to transfer the UDP-GlcUA and the UDP-GalNAc, respectively. It is also known that a mutant obtained by replacing an amino acid in one of the active sites for sugar transfer with another amino acid shows only a sugar transfer activity of the other sugar transfer site (Patent Document 2).
In addition, it is known that an HA oligosaccharide can be produced by using a point mutant of an HA synthase derived from *Pasteurella multocida* which is an animal-infectious bacterium, but production of a CH oligosaccharide and the like is not disclosed (Non-patent Document 2).
Patent Document 1: JP 2003-199583 A
Patent Document 2: JP 2005-65565 A
Non-patent Document 1: Ninomiya, T. et al., 2002, Journal of Biological Chemistry, Volume 277, No. 24, p. 21567-21575
Non-patent Document 2: Paul, L. DeAngelis et al., 2003, Journal of Biological Chemistry, Volume 278, No. 37, p. 35199-35203

### DISCLOSURE OF THE INVENTION

### Problem to be solved by the Invention

An object of the present invention is to provide a method of producing a CH with a desired sugar chain length, a method of producing a fraction containing a CH with a substantially single sugar chain length, and the like.

### Means for solving the Problems

The inventors of the present invention intensively studied to solve the above-mentioned problems. As a result, the inventors have provided a method for producing a CH with a desired sugar chain length, a method for producing a fraction containing a CH with a substantially single sugar chain length, and the like by performing multiple specific steps alternately using a specific glycosyltransferase and the like, thereby the present invention has been completed.

That is, the present invention provides a method for producing a CH with a desired sugar chain length (hereinafter referred to as "Method 1 of the present invention") comprising the steps of (a) and (b):
Step (a) : a step of allowing a receptor substrate having a GlcUA residue at a non-reducing end thereof (a sugar chain obtained by the step (b) when the step (a) is performed after the step (b)), a GalNAc donor, and a GalNAc transferase to coexist in a reaction system; and
Step (b) : a step of allowing a receptor substrate having a GalNAc residue at a non-reducing end thereof (a sugar chain obtained by the step (a) when the step (b) is performed after the step (a)), a GlcUA donor, and a GlcUA transferase to coexist in a reaction system, and the steps of (a) and (b) are performed alternately.

The present invention also provides a method for producing a fraction containing a CH with a substantially single sugar chain length (hereinafter referred to as "Method 2 of the present invention") comprising the steps of (a) and (b):
Step (a): a step of allowing a receptor substrate with a substantially single sugar chain length, the substrate having a GlcUA residue at a non-reducing end thereof (a sugar chain obtained by the step (b) when the step (a) is performed after the step (b)), a GalNAc donor, and a GalNAc transferase to coexist in a reaction system; and
Step (b): a step of allowing a receptor substrate with a substantially single sugar chain length, the substrate having a GalNAc residue at a non-reducing end thereof (a sugar chain obtained by the step (a) when the step (b) is performed after the step (a)), a GlcUA donor, and a GlcUA transferase to coexist in a reaction system, and the steps of (a) and (b) are performed alternately.

The present invention also provides a method for producing a CH with a sugar chain length longer by one sugar than that of a receptor substrate (hereinafter referred to as "Method 3 of the present invention") comprising only one of the steps (a) and (b) as defined in Method 1 of the present invention.
Hereinafter, Methods 1 to 3 of the present invention is together referred to as "the method of the present invention".

Each of the GalNAc transferase in the step (a) and the GlcUA transferase in the step (b) of the method of the present invention is preferably an enzyme shown in the following (A).
(A) an enzyme comprising the amino acid sequence shown in SEQ ID NO: 2
   Moreover, it is also preferable that the GalNAc transferase in the step (a) of the method of the present invention is an enzyme shown in the following (B), and the GlcUA transferase in the step (b) of the method of the present invention is an enzyme shown in the following (C).
(B) a mutant of the enzyme comprising the amino acid sequence shown in SEQ ID NO: 2, in which one to several amino acids in a region shown by amino acid numbers 435 to 539 in SEQ ID NO: 2 are replaced by another amino acid.
(C) a mutant of the enzyme comprising the amino acid sequence shown in SEQ ID NO: 2, in which one to several amino acids in a region shown by amino acid numbers 153 to 258 in SEQ ID NO: 2 are replaced by another amino acid.

Of these, a mutant of the enzyme, in which a "region shown by amino acid numbers 435 to 539" is a "region shown by amino acid numbers 519 to 521", a "region shown by amino acid numbers 153 to 258" is a "region shown by amino acid numbers 239 to 241", and "one to several" is "1 to 3", is preferable. Further, a mutant of the enzyme, in which only an amino acid of amino acid number 521 is replaced by another amino acid, and only an amino acid of amino acid number 241 is replaced by another amino acid, is more preferable.

It is also preferable that the method of the present invention further comprises a step of removing at least one of the transferase and the donor which are allowed to coexist in each of the steps (a) and (b) soon after each of the steps. In this case, it is preferable to remove only the transferase.
In addition, it is also preferable that the GalNAc donor used in the method of the present invention is UDP-GalNAc, and the GlcUA donor used in the method of the present invention is UDP-GlcUA.

"Coexistence" in the method of the present invention is performed preferably for 10 minutes to 24 hours under a condition of 10 to 50°C, more preferably for 30 minutes to 5 hours under a condition of 20 to 40°C, and still more preferably for 1 to 4 hours under a condition of 25 to 37°C.
Further, each of the transferases which are allowed to coexist in each of the steps (a) and (b) in the method of the present invention is preferably immobilized on a carrier.
Besides, the receptor substrate used in the method of the present invention is preferably a CH or its derivative. The derivative of the CH is preferably a CH to which PA is bound via a covalent bond.

### Effects of the Invention

The method of the present invention is very useful, because the method easily produces a CH with a desired sugar chain length and a fraction containing a CH with a substantially single sugar chain length at a low cost in an industrial scale, without substantially producing by-products.
In addition, the CH with a desired sugar chain length, the fraction containing a CH with a substantially single sugar chain length, and the like, which are produced by the present invention, are very useful substances for searching a physiological activity and the like depending on a change of a sugar chain, which has not been explored so far. The substances are also useful for materials for a pharmaceutical, food and beverage, cosmetics, and the like.

### Best Mode for carrying out the Invention

The present invention will be hereinafter described by the best mode for carrying out the invention.
<1> Method 1 of the present invention
   Method 1 of the present invention is a method for producing a CH with a desired sugar chain length comprising the steps of (a) and (b) described below and performing these steps alternately.
   Step (a) : a step of allowing a receptor substrate having a GlcUA residue at its non-reducing end (a sugar chain obtained by the step (b) when the step (a) is performed after the step (b)), a GalNAc donor, and a GalNAc transferase to coexist in a reaction system.
   Step (b) : a step of allowing a receptor substrate having a GalNAc residue at its non-reducing end (a sugar chain obtained by the step (a) when the step (b) is performed after the step (a)), a GlcUA donor, and a GlcUA transferase to coexist in a reaction system.

Method 1 of the present invention can produce a CH while controlling an increase of a sugar chain length, so that a CH with a desired sugar chain length can be easily produced. Therefore, Method 1 of the present invention also includes a concept of a method of controlling (managing) an increase of a CH sugar chain length and the like. It should be noted that "CH", which is an object (object in which an increase of a CH sugar chain length is controlled) produced by Method 1 of the present invention, includes not only an original CH composed of a repeating unit of GlcUA-GalNAc alone but also derivatives of CH oligosaccharide and CH and the like.

The step (a) includes a step of allowing a receptor substrate having a GlcUA residue at its non-reducing end (a sugar chain obtained by the step (b) when the step (a) is performed after the step (b)), a GalNAc donor, and a GalNAc transferase to coexist. That is, the step (a) includes: transferring a GalNAc residue of the GalNAc donor to a GlcUA residue of the receptor substrate having a GlcUA residue at its non-reducing end by a GalNAc transferase as a reaction enzyme to produce a sugar chain which has a repeating unit of GlcUA-GalNAc as a non-reducing end and has a saccharide residue number longer by one more residue than the receptor substrate.

On the other hand, the step (b) includes a step of allowing a receptor substrate having a GalNAc residue at its non-reducing end (a sugar chain obtained by the step (a) when the step (b) is performed after the step (a)), a GlcUA donor, and a GlcUA transferase to coexist. That is, the step (b) includes: transferring a GlcUA residue of the GlcUA donor to a GalNAc residue of the receptor substrate having a GalNAc residue at its non-reducing end by a GlcUA transferase as a reaction enzyme to produce a sugar chain that has the repeating unit of GalNAc-GlcUA as a non-reducing end and has a saccharide residue number longer by one residue than the receptor substrate.

A "receptor substrate having a GlcUA residue at its non-reducing end" herein described is not particularly limited as long as the receptor substrate is a sugar chain (including its derivative) having a GlcUA residue at its non-reducing end. Further, a "receptor substrate having a GalNAc residue at its non-reducing end" is not particularly limited as long as the receptor substrate is a sugar chain (including its derivative) having a GalNAc residue at its non-reducing end.

As these receptor substrates, sugar chains each represented by the following general formulae (1) and (2) may be exemplified.

GlcUA-GalNAc-R¹ (1)

GalNAc-GlcUA-R² (2)

where - represents a glycoside linkage and R¹ and R² each represent an optional group, which are the same or different from each other.

As "R¹" and "R²", a sugar chain residue having a CH skeleton and a sugar chain residue having an HA skeletonmay be exemplified. Examples of the "sugar chain residue having a CH skeleton" herein described include a CH residue and a CS residue. These sugar chain residues may be further bound with another chemical substance or the like.
In the method of the present invention, "GlcUA" and "GalNAc" are preferably D-GlcUA and D-GalNAc, respectively. The glycoside linkage between GlcUA and GalNAc (GlcUA-GalNAc) is preferably a β1-3 linkage, and the glycoside linkage between GalNAc and GlcUA (GalNAc-GlcUA) is preferably a β1-4 linkage.

A sugar chain size of the receptor substrate is not particularly limited, and may also range from a high molecular weight polysaccharide chain to an oligosaccharide having about 1 to 20 saccharides. Specific examples of the "receptor substrate having a GlcUA residue at its non-reducing end" include CH oligosaccharides such as CH disaccharide, CH trisaccharide, CH tetrasaccharide, CH pentasaccharide, CH hexasaccharide, CH heptasaccharide, CH octosaccharide, CH nonasaccharide, CH decasaccharide and a high molecular weight CH as a sugar chain in the formula (1), and a sugar chain represented by the general formula (1) having CS oligosaccharides such as CS disaccharide, CS trisaccharide, CS tetrasaccharide, CS pentasaccharide, CS hexasaccharide, CS heptasaccharide, CS octosaccharide, CS nonasaccharide, CS decasaccharide and a high molecular weight CS as R¹, and having HA oligosaccharides such as HA disaccharide, HA trisaccharide, HA tetrasaccharide, HA pentasaccharide, HA hexasaccharide, HA heptasaccharide, HA octosaccharide, HA nonasaccharide, HA decasaccharide and a high molecular weight HA asR¹. Specific examples of the "receptor substrate having a GalNAc residue at its non-reducing end" include CH oligosaccharides such as CH disaccharide, CH trisaccharide, CH tetrasaccharide, CH pentasaccharide, CH hexasaccharide, CH heptasaccharide, CH octosaccharide, CH nonasaccharide, CH decasaccharide and a high molecular weight CH as a sugar chain represented by the formula (2), anda sugar chain represented by the general formula (2) having CS oligosaccharides such as CS disaccharide, CS trisaccharide, CS tetrasaccharide, CS pentasaccharide, CS hexasaccharide, CS heptasaccharide, CS octosaccharide, CS nonasaccharide, CS decasaccharide and a high molecular weight CS as R², and having HA oligosaccharides such as HA disaccharide, HA trisaccharide, HA tetrasaccharide, HA pentasaccharide, HA hexasaccharide, HA heptasaccharide, HA octosaccharide, HA nonasaccharide, HA decasaccharide and a high molecular weight HA as R².

These receptor substrates can be produced by a known method, and also commercially available. In addition, as derivatives of these sugar chains, derivatives obtained by binding other chemical substances to these sugar chains can be exemplified. Kinds and the like of the other chemical substances that binds to these sugar chains are not particularly limited. As these chemical substances, PA is exemplified. Manners of binding of the chemical substances to the sugar chains are not limited. Examples thereof include a covalent bond. Of these, a manner in which PA is bound to a sugar chain via a covalent bond is preferable. A manner in which PA is bound to a reducing end of a sugar chain is more preferable. A manner in which PA is bound to a reducing end of a CH (including oligosaccharides of CH) via a covalent bond is most preferable. A sugar chain to which PA is bound via a covalent bond can be prepared by a method of amino-alkylating PA to bind to a reducing end of the sugar chain using a reductant. As the reductant, cyanoborohydride, a dimethylamino borane complex, a trimethylamino borane complex, or the like is preferably used. A method using a trimethylamino borane complex is particularly preferable.

In Method 1 of the present invention, one kind of sugar chain with a desired sugar chain length can be produced by using a receptor substrate composed of one kind of compound as such a receptor substrate. Alternatively, by using a plural kinds (preferably 2 to 5 kinds and more preferably 2 to 3 kinds) of receptor substrates, a plurality of sugar chains each containing desired sugar chain lengths derived from each of the receptor substrates may be produced. When the production of only CH decasaccharide (sugar chain length: 10 sugars) is desired, for example, one kind of CH oligosaccharide having a shorter sugar chain length than that of decasaccharide may be used as a receptor substrate. When a mixture of a CH octasaccharide (sugar chain length: 8 sugars) and a CH dodecasaccharide (sugar chain length: 12 sugars) is simultaneously produced, for example, CH oligosaccharides each having chain lengths of (8-n) sugars and (12-n) sugars (n represents an integer of 1 or more) may be used as receptor substrates.

Method 1 of the present invention is characterized by performing the steps (a) and (b) alternately. Therefore, a "receptor substrate having a GlcUA residue at its non-reducing end" in the step (a) means a "sugar chain obtained by the step (b) " when the step (a) is performed after the step (b). Similarly, a "receptor substrate having a GalNAc residue at its non-reducing end" in the step (b) means a "sugar chain obtained by the step (a) "when the step (b) is performed after the step (a) .
A "GlcUA donor" used in Method 1 of the present invention is not limited as long as the donor is a molecule capable of donating a GlcUA residue to a sugar chain molecule, and a GlcUA nucleotide is preferable. As the GlcUA nucleotide, UDP-GlcUA, and dTDP (deoxythymidine5'-diphosphate)-GlcUA can be exemplified, and the UDP-GlcUA is preferable.

A "GalNAc donor" used in Method 1 of the present invention is not limited as long as the donor is a molecule capable of donating a GalNAc residue to a sugar chain molecule, and a GalNAc nucleotide is preferable. As the GalNAc nucleotide, and UDP-GalNAc, dTDP (deoxythymidine5'-diphosphate)-GalNAc can be exemplified, and the UDP-GalNAc is preferable.

These sugar nucleotides may be produced by a known method, or commercially available.
In addition, a "GalNAc transferase" used in Method 1 of the present invention is not limited as long as the enzyme transfers GalNAc, and an enzyme which does not substantially transfer GlcUA is preferable. Further, a "GlcUA transferase" used in Method 1 of the present invention is not limited as long as the enzyme transfers GlcUA, and an enzyme which does not substantially transfer GalNAc is preferable. Both transferases may be the same kind of enzyme or different kinds of enzymes.
When the same kind of enzyme is used as both of the transferases, the enzyme shown in the following (A) is preferable.
(A) an enzyme comprising the amino acid sequence shown in SEQ ID NO: 2.

On the other hand, when different kinds of enzymes are used as the enzymes, it is preferable that a GalNAc transferase in the step (a) of the method of the present invention is an enzyme shown in the following (B), and a GlcUA transferase in the step (B) is an enzyme shown in the following (C).
(B) a mutant of the enzyme comprising the amino acid sequence shown in SEQ ID NO: 2, wherein one to several amino acids in the region shown by amino acid numbers 435 to 539 in SEQ ID NO: 2 are replaced by another amino acid.
(C) a mutant of the enzyme comprising the amino acid sequence shown in SEQ ID NO: 2, wherein one to several amino acids in the region shown by amino acid numbers 153 to 258 in SEQ ID NO: 2 are replaced by another amino acid.
   SEQ ID NO: 1 shows the DNA sequence which is derived from *Escherichia coli* and encodes the amino acid sequence of SEQ ID NO: 2, together with the amino acid sequence.

Of these, preferable enzymes are enzymes in which "the region of amino acid numbers 435 to 539" is "the region of amino acid numbers 519 to 521", "the region of amino acid numbers 153 to 258" is "the region of amino acid numbers 239 to 241", and "one to several" is "1 to 3". Among these enzymes, an enzyme in which only the amino acid of amino acid number 521 is replaced by another amino acid, and only the amino acid of amino acid number 241 is replaced by another amino acid is preferable.
Modes of amino acid substitution, such as amino acid substitutions in the region described above, the number of amino acid substitutions, and kinds of "another amino acid" described above are not limited as long as the enzyme after substitution maintains desired enzymatic activity. "Another amino acid" described above may be selected from natural amino acids and nonnatural amino acids.

When substitution of amino acids is performed by genetic engineering, "another amino acid" described above is preferably selected from the natural amino acids (glycine, L-alanine, L-valine, L-leucine, L-isoleucine, L-serine, L-threonine, L-aspartic acid, L-glutamic acid, L-asparagine, L-glutamine, L-cysteine, L-methionine, L-lysine, L-arginine, L-histidine, L-phenylalanine, L-tyrosine, L-tryptophan, and L-proline).

Particularly, in the case where only the amino acid of amino acid number 521 in SEQ ID NO: 2 (L-aspartic acid) is replaced by another amino acid and also in the case where only the amino acid of amino acid number 241 in SEQ ID NO: 2 (L-aspartic acid) is replaced by another amino acid, "another amino acid" is preferably L-lysine. Hereinafter, an enzyme obtained by replacing only the amino acid of amino acid number 521 in SEQ ID NO: 2 (L-aspartic acid) wit L-lysine may be referred to as "D521K". An enzyme obtained by replacing only the amino acid of amino acid number 241 in SEQ ID NO: 2 (L-aspartic acid) with L-lysine may be referred to as "D241K".

"D521K" and "D241K" can be obtained by the method described in the above-mentioned Non-patent Document 2. The "D241K" is an enzyme which transfers GlcUA to a non-reducing end of a receptor substrate to form a glycoside linkage by reacting a CH having a GalNAc residue at its non-reducing end as a receptor substrate with a GlcUA nucleotide (such as UDP-GlcUA) as a donor substrate, and is an enzyme (a mutant) which substantially lacks an activity of transferring GalNAc. The "D521K" is an enzyme which transfers GalNAc to a non-reducing end of a receptor substrate to form a glycoside linkage by reacting a CH having a GlcUA residue at its non-reducing end as a receptor substrate with a GalNAc nucleotide (such as UDP-GalNAc) serving as a donor substrate, and is an enzyme (a mutant) which substantially lacks an activity of transferring GlcUA.
These enzymes may be used as a free enzyme or may be used in a state of being immobilized on a carrier (a state of an immobilized enzyme).

In the steps (a) and (b), each of a method and a condition in which a receptor substrate, a sugar donor, and a glycosyltransferase are allowed to coexist is not limited as long as the molecules thereof are brought into contact with each other to thereby allow the glycosyltransferase to act on the receptor substrate and the sugar donor. Molecular weights and the like of them at the time of co-existence can be suitably set by those skilled in the art depending on the purpose and the like.
For example, "co-existence" is preferably performed at around a neutral pH (such as a pH of about 6.5 to 8.5), and is more preferably performed in a buffer solution having a buffer action at the pH. In addition, as a temperature and a time suitable for "co-existence", there are exemplified: for 10 minutes to 24 hours under a condition of 10 to 50°C; for 30minutes to 5 hours under a condition of 20 to 40°C; and for 1 to 4 hours under a condition of 25 to 37°C. In general, the longer the desired sugar chain is, the longer contact time is required, and another peak is sometimes observed. However, this problem can be solved by further extending the contact time.

The amounts of the glycosyltransf erase and the receptor substrate at the time of "co-existence" is preferably set to about 1 nmol to 1 mmol, more preferably about 100 nmol of the receptor substrate with respect to 90 µl of the glycosyltransferase. Further, at the time of "co-existence", the sugar donor may be set to 1 equivalent or more with respect to the receptor substrate. It is more preferable that the sugar donor is allowed to exist at 1.5 to 30 equivalents with respect to the receptor substrate to perform a complete reaction of the glycosyltransferase. It is more preferable that the sugar donor is allowed to exist at 2 to 10 equivalents with respect to the receptor substrate.
Alternatively, "co-existence" may be also performed by: immobilizing the glycosyltransferase on a suitable carrier (such as beads, an ultrafiltration membrane, and a dialysis membrane); and continuously bringing the carrier into contact with a solution containing the sugar donor and the receptor substrate. Therefore, a column-type reactor, a membrane-type reactor, or the like can be adopted. Further, the method shown in WO 00/27437 can be also used, in which a receptor substrate is immobilized on a carrier to perform a transferase reaction. Still further, a bioreactor or the like which regenerates (synthesizes) a sugar donor may be used in combination.

Method 1 of the present invention may further comprise other steps as long as it comprises at least both of the steps (a) and (b). For example, it is preferable that Method 1 of the present invention further comprises a step of removing at least one of the transferase and the donor being allowed to coexist in each of the steps (a) and (b) soon after the steps (a) and (b) in Method 1 of the present invention has been performed. In this case, it is preferable that only the transferase is removed.

The term "remove" in Method 1 of the present invention means to remove an action of a molecule from the co-existence system. Therefore, the term "remove" includes, needless to say, physically removing molecules themselves, and also includes a mode in which the action of the molecule is removed by deactivating the molecules.
A method of removing at least one of a transferase and a donor is not particularly limited. In the case of removing the transferase, which is immobilized on a solid phase, the solid phase is removed by a proper method, thereby the trasferase can be removed. The donor can be removed by using, for example, gel filtration chromatography or an ultrafiltration membrane.

However, the reaction for producing a sugar chain with a sugar residue number longer by one residue than that of the receptor substrate in the steps (a) and (b) of Method 1 of the present invention can be controlled by reaction conditions (such as a reaction time and the like) as described in the examples shown below, so it is not essential to remove at least one of the transferase and the donor that were allowed to coexist in each of the steps so as to perform each reaction independently.
Therefore, the degree of "removal" is not necessarily 100%, and the degree can be suitably set by those skilled in the art depending on the purpose and the like.

For removal of the glycosyltransferase, for example, the glycosyltransferase may be deactivated by heat. When the glycosyltransferase is immobilized on a carrier, the removal may be performed by physically removing the carrier. The physical removal of the carrier may be easily performed by using a filtration membrane or the like or conducting centrifugation or the like.

Furthermore, it is needless to say that Method 1 of the present invention may further comprise a step of purifying the CH (with a desired sugar chain length) obtained by Method 1 of the present invention and a step of performing quality check.
Method 1 of the present invention is characterized by performing the steps (a) and (b) alternately. Therefore, the steps (a) and (b) may be performed alternately starting from the step (a) (for example, step (a), step (b), step (a), step (b), ...), and may be performed alternately starting from the step (b) (for example, step (b), step (a), step (b), step (a), ...). It should be noted that the term "alternately" also includes a case where the reaction is terminated only by performing two steps (for example, a case where a set of step (a) and step (b) is performed, and the reaction is finished and a case where a set of step (b) and step (a) is performed, and the reaction is finished).
One of the step (a) and step (b) is performed once, thereby a sugar chain is extended by one saccharide. Therefore, a CH with a desired sugar chain length can be produced by appropriately setting the number of each of the steps "alternately" performed. Those skilled in the art can suitably set and control the number of each of the steps to be alternately performed on the basis of the condition of co-existence of each molecule in each of the steps described above.
A product produced by Method 1 of the present invention (a CH with a desired sugar chain length) may be in the state of a solution or in the state of a solid (such as a powder or a frozen solution).

### <2> Method 2 of the present invention

Method 2 of the present invention is a method for producing a fraction containing a CH with a substantially single sugar chain length, which comprises the steps (a) and (B) described below and these steps are performed alternately:
step (a): a step of allowing a receptor substrate with a substantially single sugar chain length having a GlcUA residue at its non-reducing end (a sugar chain obtained by the step (b) when the step (a) is performed after the step (b)), a GalNAc donor, and a GalNAc transferase to coexist; and
step (b): a step of allowing a receptor substrate with a substantially single sugar chain length having a GalNAc residue at its non-reducing end (a sugar chain obtained by the step (a) when the step (b) is performed after the step (a)), a GlcUA donor, and a GlcUA transferase to coexist.

Method 2 of the present invention is a method for producing a fraction containing a CH with a substantially single sugar chain length, and can be performed by "using only one kind" of the "receptor substrates" in Method 1 of the present invention.
Therefore, Method 2 of the present invention is the same as Method 1 of the present invention except that the target of production is "a fraction containing a CH with a substantially single sugar chain length", and except that each of a receptor substrate having a GlcUA residue at its non-reducing end and a receptor substrate having a GalNAc residue at its non-reducing end has "substantially single sugar chain length". Thus, a reaction, an enzyme, and reactants themselves such as a substrate having a GlcUA residue at its non-reducing end and a substrate having a GalNAc residue at its non-reducing end used in Method 2 of the present invention are the same as those used in Method 1 of the present invention.

Here, the term "substantially single" means that when a fraction is analyzed by MALDI-TOF-MS, the height of a peak derived from a sugar chain with a specific sugar chain length accounts for 70% or more of the sum of the heights of the total peaks including that peak. Analysis conditions by MALDI-TOF-MS are shown in Examples described below.

In a reaction for producing a sugar chain with a sugar residue number longer by one residue than that of a receptor substrate in the steps (a) and (b) of Method 2 of the present invention, it is not essential to remove at least one of the transferase and the donor that were allowed to coexist in each of the steps so as to perform each reaction independently, similarly as in Method 1 of the present invention. It is preferable that each of the reactions is performed after at least one of the transferase and the donor is removed substantially 100%. The method of removing at least one of the transferase and the donor is the same as Method 1 of the present invention.

Here, the term "removed substantially 100%" means that when a step of producing a sugar chain with a sugar residue number longer by only one residue is performed after the transferase or the substrate is removed, at least one of the transferase and the donor is removed to the extent that the peak of a sugar chain with a sugar residue number longer by only one residue is not substantially detected by MALDI-TOF-MS (substantially neglectable). For example, "removed substantially 100%" includes the condition shown in Example 3 (2) where D521K is not used (in this case, the transferase is removed), and the peak of PA-CH7 is not substantially detected (substantially neglectable), specifically, in the condition, PA-CH6 (100 nmol) is dissolved in a Tris-HCl buffer solution containing 1.2 mM of 20 mM manganese chloride and 150 mM NaCl, then 10 µl of 50 mM MUDP-GalNAc is added thereto, and the mixture is shaken at 30°C for 2 hours, followed by purification and freeze-drying to obtain a fraction, which was then subjected to MALDI-TOF-MS.

### <3> Method 3 of the present invention

Method 3 of the present invention is a method for producing a CH with a sugar chain length longer by one than that of a receptor substrate, and comprises only one of the steps (a) and (b) described in Method 1 of the present invention.
Method 3 of the present invention is a method for producing a CH with a sugar chain length longer by one than that of a receptor substrate, in which only one of the steps (a) and (b) in Method 1 of the present invention is performed without alternately performing the steps (a) and (b).

Therefore, Method 3 of the present invention is the same as Method 1 of the present invention except that the target of production is "a CH with a sugar chain length longer by one sugar than that of a receptor substrate", and except that only one of the steps (a) and (b) in Method 1 of the present invention is performed. Thus, a reaction, an enzyme, a reactant, and the like in each step are the same as in Method 1 of the present invention.

Hereinafter, the present invention will be described in detail more specifically by referring to Examples.

### (Analysis method)

A CH and its derivatives obtained by Examples below were subj ected to MALDI-TOF-MS (AutoFlex manufactured by Bruker Daltonics) for a structural analysis. The analysis was performed in a negative mode for detecting a generated anion, and the CH and its derivatives were analyzed in a reflection mode.

### (Preparation of target)

1 µl of the obtained specimen (containing 20 to 100 pmole of CH) and 10 mg/ml of DHB (2,5-dihydroxy-benzoic acid) were mixed in 1 µl of 50% acetonitrile solution. 1 µl of the resultant mixture was spotted on a target plate, followed by drying by immediately blowing a nitrogen gas.

### Example 1

### Immobilization of the enzyme

To NHS-activated Sepharose Beads (manufactured by Amersham Biosciences Co. , Ltd., 0.1 ml), 1 ml of a physiologic phosphate buffer solution (pH of 7.2) (hereinafter referred to as "PBS") containing 0.5 mg of D241K or D521K (each obtained according to the examples of JP 2005-65565 A) and glycerol (final concentration of 20%) were added, followed by shaking at 4°C for 6 hours. After the reaction, 4 µl of 1 M ethanol amine was added thereto, and followed by shaking at 4°C for 1 hour. Then, washing was performed sequentially with 20 mM Tris-HCl buffer solution (pH of 8.0) three times, with 20 mM sodium acetate buffer solution (pH of 4.0) three times, and with 20% glycerol-PBS three times to thereby the immobilized enzymes for each of D241K and D521K were prepared. The obtained immobilized enzymes were suspended with 20% glycerol-PBS and then stored at 4°C.

By using 10 µl each of the obtained immobilized enzymes together with CH hexasaccharide and UDP- [³H] GalNAc as substrates, GalNAc transfer activity was measured. By using 10 µl each of the obtained immobilized enzymes together with CH heptasaccharide and UDP-[14C]GlcUA as substrates, GlcUA transfer activity was measured. As a result, it was confirmed that the immobilized enzyme D241K (hereinafter referred to as D241K beads) retained only GlcUA transfer activity, and the immobilized enzyme D521K (hereinafter referred to as D521K beads) only retained GalNAc transfer activity.

### Example 2

### Production of CH oligosaccharide to which PA is bound by covalent bond (pyridylaminated)

### (1) Production of CH6 (CH hexasaccharide) and CH7 (heptasaccharide)

Hyaluronidase derived from sheep testis (Sigma-Aldrich Corporation) was added to a CH obtained by chemical desulfation of CS (manufactured by SEIKAGAKU CORPORATION) to perform limited degradation of the CH in a sodium acetate buffer solution containing NaCl, thereby oligosaccharides composed of even number saccharides and having a GlcUA residue at its non-reducing end were obtained. The obtained oligosaccharides were purified by gel filtration and iron-exchange column to collect a fraction corresponding to CH6. The obtained fraction was freeze-dried. The obtained fraction was subjected to an uronic acid content analysis (carbazole method), HPLC (GPC), MALDI-TOF-MS, a disaccharide analysis after chondroitinase treatment. As a result, it was confirmed that the obtained fraction was hexasaccharide having a GalNAc residue at its reducing end and a GlcUA residue at its non-reducing end.

Furthermore, β-glucuronidase (SIGMA) was added to a mixture of CH oligosaccharides composed of multiple kinds of even number saccharides obtained by subjecting a CH to a hyaluronidase treatment in the same manner as described above. The mixture was left standing at 37°C for 18 hours in a sodium acetate buffer solution containing NaCl. The reaction solution was subjected to a purification treatment in the same manner as that described above. A fraction corresponding to CH7 was collected and then freeze-dried. The obtained fraction was subjected to the same analyses described above. As a result, it was confirmed that the obtained fraction was CH7 having GalNAc at its reducing end and GalNAc at its non-reducing end.

### (2) Production of CH6 to which PA is bound by covalent bond (PA-CH6)

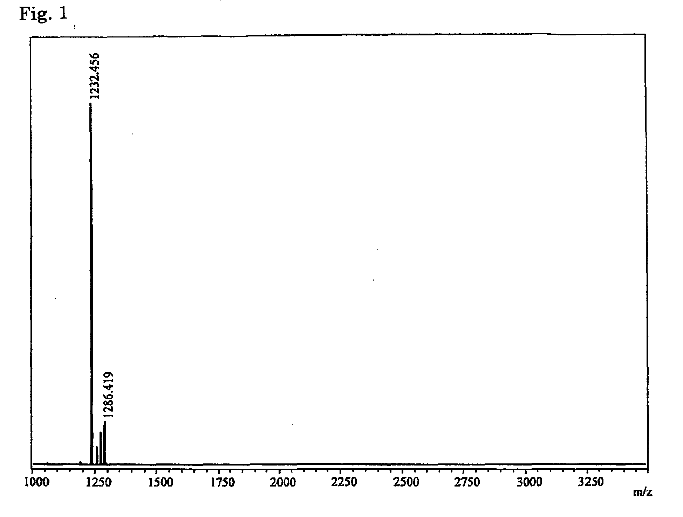

The CH6 (10 mg) obtained by the procedure (1) was dissolved in 1 ml of water, and 1 ml of PA aqueous solution adjusted to pH of 5.6 with hydrochloric acid and 1 ml of methanol solution of trimethyl amino borane complex (Aldrich) were added thereto, followed by reaction at 70°C for 3 days under sealing. The reaction solution was concentrated under reduced pressure, and freeze-dried, which was then dissolved in 0.1 ml of water again. Subsequently, the solution was passed through Dowex 50 WX8 cationic exchange resin (manufactured by The Dow Chemical Company), and was subjected to gel filtration chromatography (flow rate: 2 ml/minute) with Superdex 30 HR 16/60 column (Amersham) using 0.2 M ammonium acetate as a development solution. The solution to be detected was monitored with a fluorescence detector (Ex: 310 nm, Em: 370 nm), and fractions containing PA-CH6 were collected. The fractions were further subjected to ion-exchange chromatography by Linear Gradient method using 30 to 200 mM of KH₂PO₄ with SAX Magnum 9/25HPLC column (manufactured by Whatman, Inc.) for purification, thereby PA-CH6 was obtained. The PA-CH6 was analyzed in the same manner as the procedure (1) to confirm the structure and purity of the PA-CH6 (Fig. 1).

### Example 3

### Production of pyridylaminated-oligosaccharide with desired sugar chain length

### (1) Setting of reaction time

The PA-CH6 (100 nmol) obtained in Example 2 (2) was dissolved in 1.2 ml of 50 mM Tris-HCl buffer solution (pH of 7.2) containing 20 mM manganese chloride and 150 mM NaCl, and the mixture was added to a container containing the D521K beads obtained in Example 1. 10 µl of 50 mM UDP-GalNAc and 5 pmol (0.1 µ Ci) of UDP- [³H] GalNAc were added into the container. The container was shaken at 30°C, and 1 hour later, the reaction solution was subjected to separation with gel filtration chromatography using Superdex Peptide HR 10/30 column. A transfer amount of GalNAc was measured by a scintillation counter, which confirmed that the sugar transfer reaction was almost perfectly completed one hour later. Based on the result, the reaction time was set as 2 hours.

### (2) Production of PA-CH7

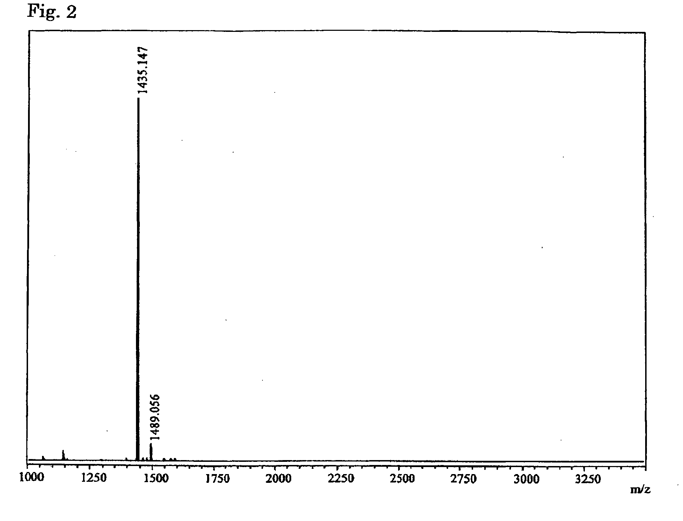

In the same manner as Example 3 (1), the PA-CH6 obtained in Example 2 (2) was dissolved in the same Tris-HCl buffer solution as described above, and the mixture was added into a container containing D521K beads obtained in Example 1. UDP-GalNAc was added into the container, followed by shaking at 30°C for 2 hours. The reaction solution (1.2 ml) was subjected to filtration with a filter (Ultra Free CL, manufactured by Millipore Corporation, pore diameter of 0.45 µm) to remove the enzyme-immobilized beads, thereby a filtrate containing PA-CH7 was obtained. The filtrate was used as it is for the following saccharide transfer reaction.
The filtrate was subjected to gel filtration chromatography using Superdex Peptide HR 10/30 column to purify PA-CH7, and the purified PA-CH7 was freeze-dried. The purified fractions were subjected to MALDI-TOF-MS analysis, which confirmed that the purified fractions were high-purity PA-CH7 (Fig. 2).

### (3) Production of PA-CH8 (pyridylaminated-CH octosaccharide)

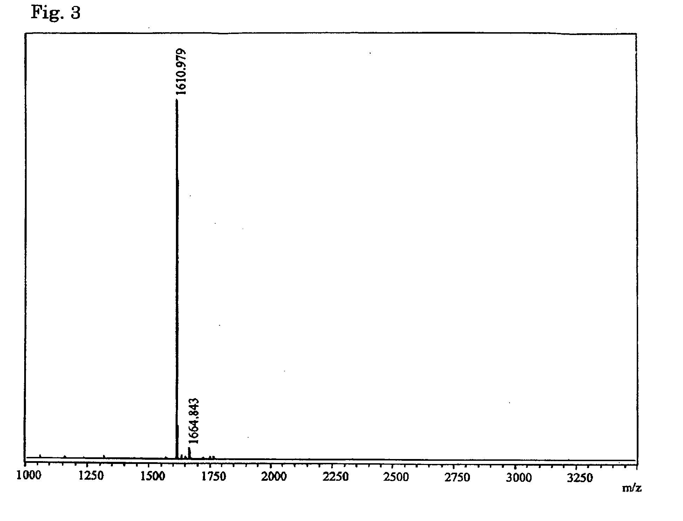

The PA-CH7 solution obtained in Example 3 (2) was added into a container containing D241K beads, and 10 µl of 50 mM UDP-GlcUA was added thereto, followed by shaking at 30°C for 2 hours. The reaction solution was treated in the same manner as the procedure (2) to remove the enzyme-immobilized beads, thereby a filtrate containing PA-CH8 was obtained. The filtrate was used as it is for producing PA-CH9 in the production of PA-CH9 to PA-CH16 (pyridylaminated-CH nonasaccharide to pyridylaminated-CH hexadecasaccharide) shown below. Purification and analysis of PA-CH8 were carried out in the same manner as described above (Fig. 3).

### Production of PA-CH9 to PA-CH16

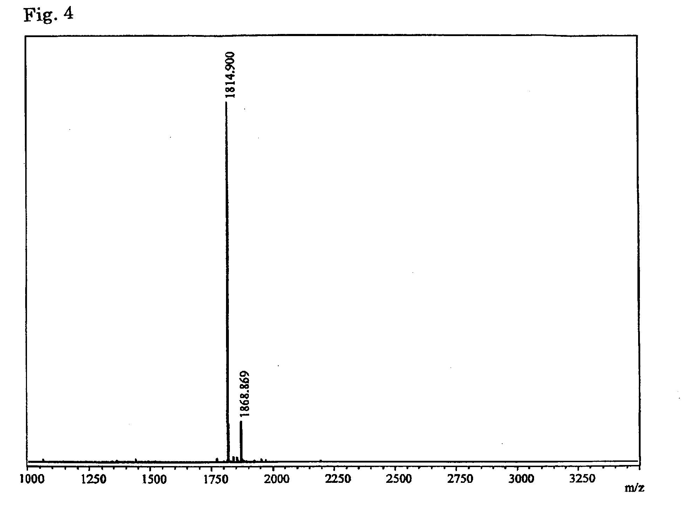

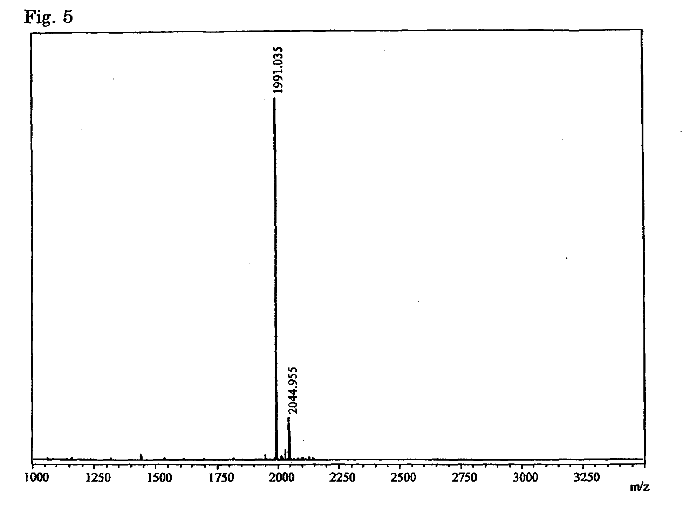

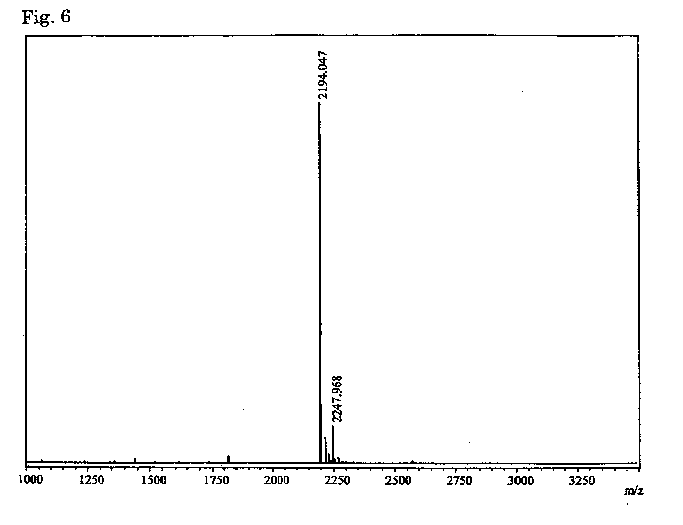

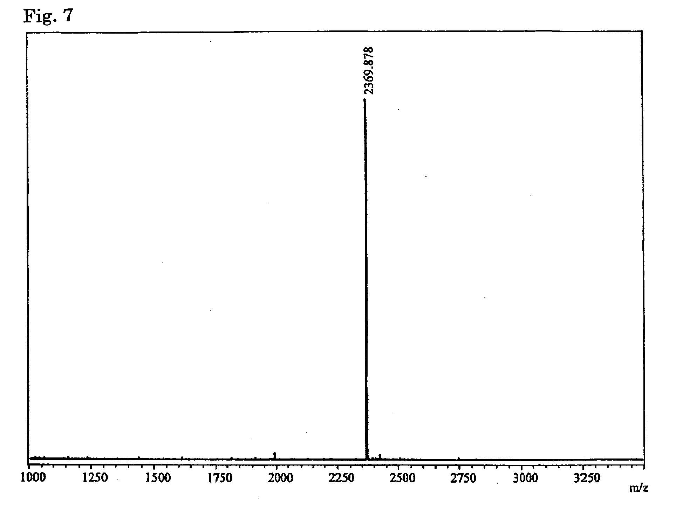

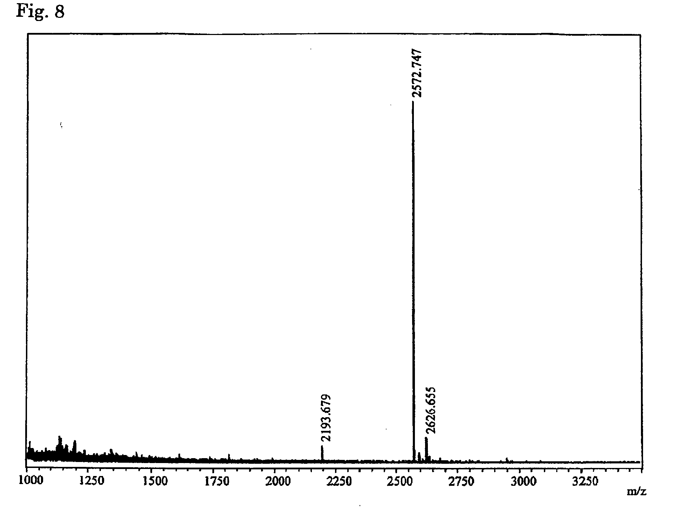

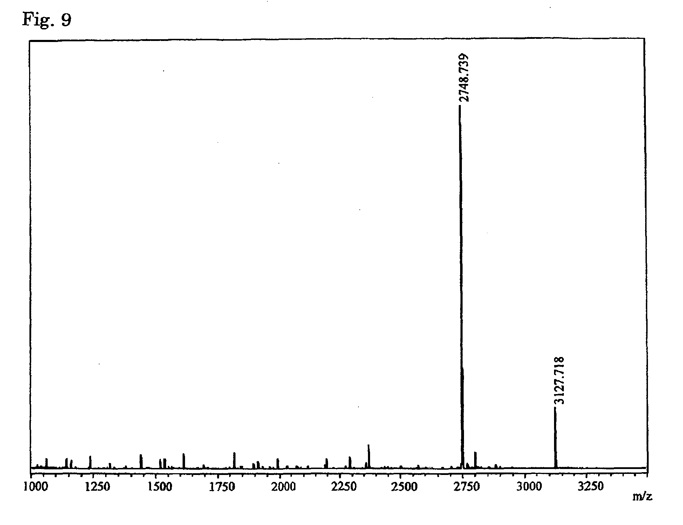

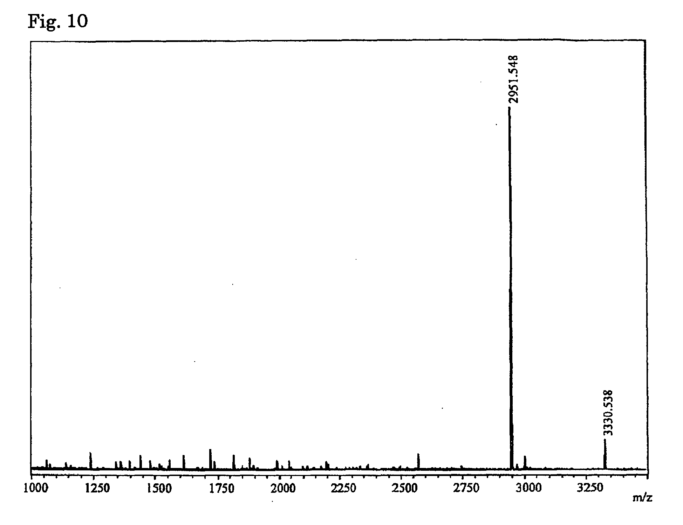

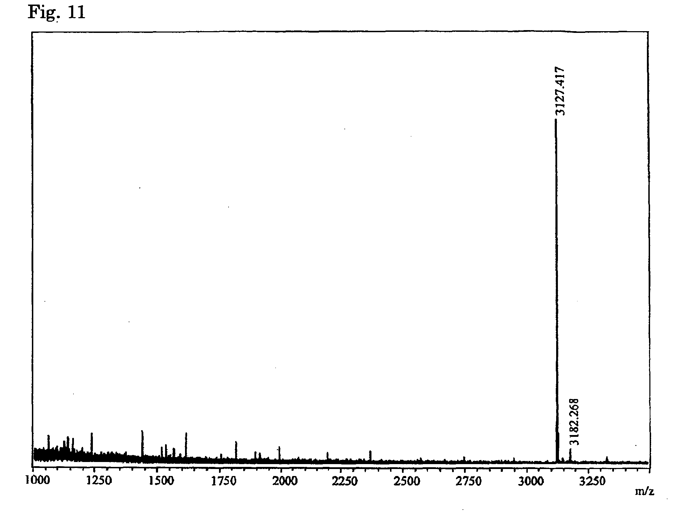

PA-CH9 was obtained by performing the same operation as in the procedure (2) using the filtrate containing the PA-CH8. PA-CH10 was obtained by performing the same operation as in the procedure (3) using the filtrate containing the PA-CH9. In the same manner as described above, the operations in the procedures (2) and (3) were repeated to thereby obtain pyridylaminated-CH oligosaccharides in which the sugar chain length was extended one by one. The thus obtained PA-CH9 to PA-CH16 were purified and analyzed in the same manner as described above (Figs. 4 to 11).

### Example 4

### Synthesis of oligosaccharides by continuous sugar chain extension

By using the CH6 obtained in Example 2 (1), the reactions were alternately performed in the same manner as in Example 3 until sugar chains with a desired sugar chain length were obtained, thereby each of CH7 to CH16 (CH heptasaccharide to CH hexadecasaccharide) was produced. Purification and analysis of CH7 to CH16 were carried out in the same manner as described above. It was confirmed that each of CH7 to CH16 was obtained.

### Brief Description of the Drawings

Fig. 1 illustrates MALDI-TOF-MS spectrum of PA-CH6.
Fig. 2 illustrates MALDI-TOF-MS spectrum of PA-CH7.
Fig. 3 illustrates MALDI-TOF-MS spectrum of PA-CH8.
Fig. 4 illustrates MALDI-TOF-MS spectrum of PA-CH9.
Fig. 5 illustrates MALDI-TOF-MS spectrum of PA-CH10.
Fig. 6 illustrates MALDI-TOF-MS spectrum of PA-CH11.
Fig. 7 illustrates MALDI-TOF-MS spectrum of PA-CH12.
Fig. 8 illustrates MALDI-TOF-MS spectrum of PA-CH13.
Fig. 9 illustrates MALDI-TOF-MS spectrum of PA-CH14.
Fig. 10 illustrates MALDI-TOF-MS spectrum of PA-CH15.
Fig. 11 illustrates MALDI-TOF-MS spectrum of PA-CH16.

## Claims

1. A method for producing a chondroitin with a desired sugar chain length, comprising the steps of (a) and (b):
(a) allowing a receptor substrate having a glucuronic acid residue at a non-reducing end thereof (a sugar chain obtained by the step (b) when the step (a) is performed after the step (b)), an N-acetylgalactosamine donor, and an N-acetylgalactosamine transferase to coexist in a reaction system; and
(b)allowing a receptor substrate having an N-acetylgalactosamine residue at a non-reducing end thereof (a sugar chain obtained by the step (a) when the step (b) is performed after the step (a)), a glucuronic acid donor, and a glucuronic acid transferase to coexist in a reaction system, and
the steps of (a) and (b) are performed alternately.

2. A method for producing a fraction containing a chondroitin with a substantially single sugar chain length, comprising the steps of (a) and (b):
(a) allowing a receptor substrate with a substantially single sugar chain length, having a glucuronic acid residue at a non-reducing end thereof (a sugar chain obtained by the step (b) when the step (a) is performed after the step (b)), an N-acetylgalactosamine donor, and an N-acetylgalactosamine transferase to coexist in a reaction system; and
(b) : allowing a receptor substrate with a substantially single sugar chain length, having an N-acetylgalactosamine residue at a non-reducing end thereof (a sugar chain obtained by the step (a) when the step (b) is performed after the step (a)), a glucuronic acid donor, and a glucuronic acid transferase to coexist in a reaction system, and the steps of (a) and (b) are performed alternately.

3. A method for producing a chondroitin with a sugar chain length longer by one sugar than that of the receptor substrate, comprising only one of the steps (a) and (b) as defined in claim 1.

4. The production method according to any one of claims 1 to 3, wherein both of the N-acetylgalactosamine transferase in the step (a) and the glucuronic acid transferase in the step (b) are an enzyme shown in the following (A):
(A) an enzyme comprising the amino acid sequence shown in SEQ ID NO: 2.

5. The production method according to any one of claims 1 to 3, wherein the N-acetylgalactosamine transferase in the step (a) is an enzyme shown in the following (B), and the glucuronic acid transferase in the step (b) is an enzyme shown in the following (C):
(B) a mutant of the enzyme comprising the amino acid sequence shown in SEQ ID NO: 2, wherein one to several amino acids in a region shown by amino acid numbers 435 to 539 in SEQ ID NO: 2 is/are replaced by other amino acid(s); and
(C) a mutant of the enzyme comprising the amino acid sequence shown in SEQ ID NO: 2, wherein one to several amino acids in a region shown by amino acid numbers 153 to 258 in SEQ ID NO: 2 is/are replaced by another amino acid(s).

6. The production method according to claim 5, wherein the "region of amino acid numbers 435 to 539" is a "region of amino acid numbers 519 to 521", the "region of amino acid numbers 153 to 258" is a "region of amino acid numbers 239 to 241", and "one to several" is "1 to 3".

7. The production method according to claim 6, wherein only the amino acid of amino acid number 521 is replaced by another amino acid, and only the amino acid of amino acid number 241 is replaced by another amino acid.

8. The production method according to any one of claims 1 to 7, further comprising
the step of removing at least one of the transferase and donor being allowed to coexist in each of the steps (a) and (b) soon after each of the steps.

9. The production method according to claim 8, wherein only the transferase is removed.

10. The production method according to any one of claims 1 to 9, wherein the N-acetylgalactosamine donor is UDP-N-acetylgalactosamine, and the glucuronic acid donor is UDP-glucuronic acid.

11. The production method according to any one of claims 1 to 10, wherein the "coexistence" is performed for 10 minutes to 24 hours under a condition of 10 to 50°C.

12. The production method according to any one of claims 1 to 11, wherein the "coexistence" is performed for 30 minutes to 5 hours under a condition of 20 to 40°C.

13. The production method according to any one of claims 1 to 12, wherein the "coexistence" is performed for 1 hour to 4 hours under a condition of 25 to 37°C.

14. The production method according to any one of claims 1 to 13, wherein each of the transferases which are allowed to coexist in each of reaction systems in the steps (a) and (b) is immobilized on a carrier.

15. The production method according to any one of claims 1 to 14, wherein the receptor substrate is a chondroitin or a chondroitin derivative.

16. The production method according to claim 15, wherein the chondroitin derivative is a chondroitin to which aminopyridine is bound via a covalent bond.
